(19) Europäisches Patentamt
European Patent Office
Office européen des brevets

(11) Publication number: **0 235 949**
**A1**

(12) **EUROPEAN PATENT APPLICATION**

(21) Application number: **87300906.2**

(22) Date of filing: **02.02.87**

(51) Int. Cl.⁴: **A 61 F 13/02**, A 61 L 15/06

(30) Priority: **18.02.86 US 830644**

(43) Date of publication of application: **09.09.87 Bulletin 87/37**

(84) Designated Contracting States: **AT BE CH DE ES FR GB IT LI NL**

(71) Applicant: **Seton Company, 849 Broadway, Newark New Jersey 07104 (US)**

(72) Inventor: **Edenbaum, Martin I., 12 Springwood Drive, Princeton Junction New Jersey 08550 (US)**
Inventor: **Rybalka, Borys, 9739 Redd Rambler Place, Philadelphia Pennsylvania 19115 (US)**

(74) Representative: **Miller, Joseph et al, J. MILLER & CO. Lincoln House 296-302 High Holborn, London WC1V 7JH (GB)**

(54) **Cohesive dressing.**

(57) A cohesive dressing, and a process for manufacturing it, which is prepared as a single sheet of self-adherent flexible foam. The foam is self-adherent due to the incorporation therein, throughout the foam matrix itself, of a limited quantity of a pressure sensitive adhesive composition. The process for manufacturing the cohesive dressing includes admixing an aqueous polymeric dispersion with an aqueous pressure sensitive adhesive emulsion until each is substantially uniformly distributed throughout the admixture, and frothing, casting or otherwise forming, curing and drying the admixture. The admixture must be frothed and cured to form a foam layer which is self-adherent without demonstrating significant wound or skin adherence. The polymeric composition of the cohesive dressing is preferably a polyurethane, and the pressure sensitive adhesive component of the foam is preferably an acrylic adhesive composition. The foam sheet thus produced may be cut or formed into drapes, tapes or dressings having any desired size or shape.

EP 0 235 949 A1

# COHESIVE DRESSING

## FIELD OF THE INVENTION

The present invention relates to dressings which are self-adherent and, more particularly, to a cohesive dressing which adheres to itself, but not to the skin or wound surface, when wrapped and overlapped into position.

## BACKGROUND OF THE INVENTION

In the treatment of the various skin wounds, such as burns, abrasions, contusions and surgical and accidental incisions, the use of adhesive tapes to secure absorbent dressing materials is a standard element of dressing technique. Unfortunately, this widespread use of adhesive tapes can cause inflammation and tissue damage or, at least, discomfort to the patient during use and removal. Indeed, tissue damage caused by the use of adhesives and adhesive tapes can be severe in the case of, for example, the burn victim or the debilitated or elderly patient.

Prior efforts to minimize the damage to the skin caused by these topical adhesives led to developments in two separate medical product areas. First, extensive research directed toward preparing "gentler" adhesives resulted in products such as the low-tack high solids polymers and the porous or otherwise discontinuous adhesive sheets, both of which are now available in medical/surgical adhesive tape form. These adhesive compositions minimized, but unfortunately did not eliminate, the adverse effects caused by adhesive tapes generally. The "interlocking" dressings made up the second product area, within which sheet materials or tape-like dressings demonstrate self-adherence commensurate with the mechanical structure of their interlocking surfaces. The best known example of this type of wound dressing is "cling gauze," whose characteristic tufted-and-looped weave enables it to cling to itself without the use of adhesives.

Cling gauze is now widely used in hospital wound care, particularly in trauma, burn and critical care units.

Notwithstanding its advantages, however, cling gauze itself provides obstacles to easy, effective wound care. In order for cling gauze to "cling," both the inner and the outer surfaces must remain free to interlock, i.e., neither the inner nor the outer surface may be coated or laminated with any other sheet material or coating. Accordingly, by its very nature, cling gauze is never waterproof or water-resistant, because it is always essentially an untreated, unlaminated gauze sheet. In addition, cling gauze is even more likely to lint than conventional gauze because the action of interlocking and separating the gauze surfaces inevitably frees large numbers of cotton fibers, thus creating dust and lint in the area of the wound. Moreover, after cling gauze has been left in place for a number of hours, the natural movement of the patient tends to tangle the interlocked structure, and cling gauze is thus frequently very difficult to remove. Finally, because cling gauze is primarily gauze and no more, it is both bacteria permeable and largely nonresilient, with the result that dressings made of cling gauze provide protection against neither contusion nor infection.

In view of the prior art, therefore, a need remains for an improved cohesive dressing which is resilient, non-linting, easy to apply and remove and self-adherent without being wound- or skin-adherent. Such a product might also demonstrate improved absorbency over known gauze-like or other fibrous materials, along with ready liquid permeability in the area of the wound. Most preferably, such a dressing would have a moisture vapor permeable, moisture and bacteria impermeable outer layer, or skin, thereon.

## BRIEF DESCRIPTION OF THE INVENTION

As an article to meet these needs, the present invention is a cohesive dressing, and a process for manufacturing it, which is prepared as a single sheet of self-adherent flexible foam. The foam is self-adherent due to the incorporation therein, throughout the foam matrix itself, of a limited

quantity of a pressure sensitive adhesive composition. The process for manufacturing the cohesive dressing includes admixing an aqueous polymeric dispersion with an aqueous pressure sensitive adhesive emulsion until each is substantially uniformly distributed throughout the admixture, and frothing, casting or otherwise forming, curing and drying the admixture. The admixture must be frothed and cured to form a foam layer which is self-adherent without demonstrating significant wound or skin adherence. The resulting flexible foam may be either hydrophobic or hydrophilic; preferably, the hydrophilic foams are readily permeable to moisture, serum (and other wound exudates) and most topical medicaments. The foam sheet optionally may be cast onto a moisture vapor permeable, moisture and bacteria impermeable polymeric skin which contains equivalent adhesive solids as does the foam. The polymeric composition of the cohesive dressing is preferably a polyurethane, and the pressure sensitive adhesive component of the foam is preferably an acrylic adhesive composition. The foam sheet thus produced may be cut or formed into drapes, tapes or dressings having any desired size or shape.

## DETAILED DESCRIPTION OF THE INVENTION

As described above, the process for preparing the present cohesive dressing includes frothing, casting, curing and drying an admixture of an aqueous polymeric composition and an aqueous pressure sensitive adhesive emulsion. The equipment and methods for accomplishing these steps are well-known in the art, as described below. Inasmuch as the frothed admixture contains two essential components, however, the cohesive dressing is best described by identifying first those polymers and adhesive resins suitable for use therein.

### I. Adhesive Emulsions

A number of adhesive emulsions are suitable for use in the present invention. The adhesive composition itself must be, simply, dispersible in water, noncytotoxic within medically acceptable limits, and co-curable

with the aqueous polymer dispersion discussed below. Suitable adhesive emulsions therefore contain, for example, acrylic adhesive resins, blends of butadiene-acrylonitrile copolymers with resins such as oil-soluble, heat-hardening phenol-formaldehyde resins, two-step thermosetting phenolic resin compositions, coumarone-indene resins, polyterpene resins and the like; polychloroprene combined with heat-hardening phenol-formaldehyde resins, rosin-phenol resins, vinyl alkyl ether polymer based adhesives, thermoplastic styrene-butadiene block polymer rubbers mixed with resins such as those described, and other such adhesive compositions.

Particularly useful pressure sensitive adhesive emulsions include the acrylic emulsions sold in association with the mark Rhoplex®, by Rohm and Haas Company. These Rhoplex® adhesives include Rhoplex N-560, Rhoplex N-580, Rhoplex N-582, Rhoplex N-619, Rhoplex N-1031 and Rhoplex LC-67. These emulsions are particularly well suited to the preparation of the present cohesive dressing because, for example, they cure without additional tackifying resins to compositions having excellent resistance to delamination under wet conditions. In addition, cured products prepared from these adhesive emulsions do not lose tack under the application of heat, i.e., up to 400$^{o}$ F.; medical products containing these adhesive compositions may therefore be autoclaved without adhesive deterioration. For these reasons, although other adhesive compositions are suitable for use in the present invention, the Rhoplex® adhesive emulsions are preferred.

## II. Polymeric Dispersions

Although a wide variety of aqueous polymeric dispersions are suitable for use in the preparation of the present cohesive dressing, cured products formed from the dispersed polymer particles ordinarily should demonstrate significant, and preferably substantial, hydrophilicity, and should be prepared with the understanding that the constituents of the dispersion should not result in medically unacceptable cytotoxicity in the ultimate composition so produced. For these reasons, therefore, the preferred aqueous polymeric dispersions for use in the present invention are those dispersions which contain fully-reacted ionically solubilized polyurethane

particles in water, without added cosolvents or emulsifiers. More specifically, these dispersions are in contrast with the emulsified isocyanate copolymers such as those disclosed in U.S. Patent No. 2,968,575, which are prepared and dispersed in water with the aid of detergents under the action of powerful shearing forces. The emulsified polyurethanes have the disadvantage that a detergent must be used to form the emulsion and such detergent is usually retained in the cured polyurethane, thus seriously detracting from the overall physical and chemical properties of the final product.

The preferred system for preparing aqueous ionic polyurethane dispersions is to prepare polymers that have free acid groups, preferably carboxylic acid groups, covalently bonded to the polymer backbone. Neutralization of these carboxyl groups with an amine, preferably a water soluble mono-amine, affords water dilutability. Careful selection of the compound bearing the carboxylic groups must be made because isocyanates, the reactive group employed most often in the generation of urethane linkages, are generally reactive with carboxylic groups. However, as disclosed in U.S. Patent No. 3,412,054, incorporated herein by reference, 2,2-hydroxymethyl-substituted carboxylic acids can be reacted with organic polyisocyanates without significant reaction between the acid and isocyanate groups as a result of the steric hindrance of the carboxyl by the adjacent alkyl groups. This approach provides the desired carboxyl-containing polymer with the carboxylic groups being neutralized with the tertiary mono-amine to provide an internal quaternary ammonium salt and, hence, water dilutability.

Suitable carboxylic acids, and, preferably, the sterically hindered carboxylic acids, are well-known and readily available. For example, they may be prepared from an aldehyde that contains at least two hydrogens in the alpha position which are reacted in the presence of a base with two equivalents of formaldehyde to form a 2,2-hydroxymethyl aldehyde. The aldehyde is then oxidized to the acid by procedures known to those skilled in the art. Such acids are represented by the structural formula:

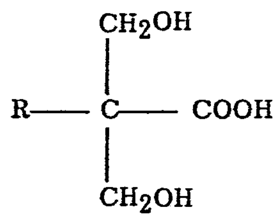

wherein R represents hydrogen, or alkyl of up to 20 carbon atoms, and preferably, up to 8 carbon atoms. A preferred acid is 2,2-di(hydroxymethyl) propionic acid.

The polymers with the pendant carboxyl groups are characterized as anionic polyurethane polymers. Further in accordance with the present invention, however, an alternate route to confer water dilutability is to use a cationic polyurethane having pendant amino groups. Such cationic polyurethanes are disclosed in U.S. Patent No. 4,066,591, incorporated herein by reference, and particularly, in Example XVIII. In the context of the present invention, however, anionic polyurethane dispersions are preferred.

The polyurethanes useful in the practice of the invention more particularly involve the reaction of di- or polyisocyanates and compounds with multiple reactive hydrogens suitable for the preparation of polyurethanes. Such diisocyanates and reactive hydrogen compounds are more fully disclosed in U.S. Patents No. 3,412,054 and No. 4,045,729. Further, the processes to prepare such polyurethanes are well recognized as exemplified by the aforementioned patents. In accordance with the present invention, therefore, aromatic, aliphatic and cycloaliphatic diisocyanates or mixtures thereof can be used in forming the polymer. Such diisocyanates, for example, are tolylene-2,4-diisocyanate; tolylene-2,6-diisocyanate; meta-phenylene diisocyanate; biphenylene-4,4'-diisocyanate; methylene-bis-(4-phenol isocyanate); 4,4-chloro-1,3-phenylene diisocyanate; naphthylene-1,5-diisocyanate; tetramethylene-1,4-diisocyanate; hexamethylene-1,6-diisocyanate; decamethylene-1,10-diisocyanate; cyclohexylene-1,4-diisocyanate; isophorone diisocyanate and the like. Preferably, the arylene and cycloaliphatic diisocyanates are used in the practice of the invention.

Characteristically, the arylene diisocyanates encompass those in which the isocyanate groups are attached to the aromatic ring. The most

preferred isocyanates are the 2,4 and 2,6 isomers of tolylene diisocyanate and mixtures thereof, due to their reactivity and ready availability. The cycloaliphatic diisocyanates used most advantageously in the practice of the present invention are 4,4'-methylene-bis(cyclohexyl isocyanate) and isophorone diisocyanate.

Selection of the aromatic or aliphatic diisocyanates is predicated upon the final end use of the particular material. As is well recognized by those skilled in the art, the aromatic isocyanates may be used where the final product is not excessively exposed to ultraviolet radiation, which tends to yellow such polymeric compositions. The aliphatic diisocyanates, on the other hand, may be more advantageously used in exterior applications and may have less tendency to yellow upon exposure to ultraviolet radiation. Although these principles form a general basis for the selection of the particular isocyanate to be used, the aromatic diisocyanates may be further stabilized by well-known ultraviolet stabilizers to enhance the final properties of the polyurethane product. In addition, antioxidants may be added in art recognized levels to improve the characteristics of the final dispersions. Typical antioxidants are the thioethers and phenolic antioxidants such as 4,4'-butylidine-bis-meta cresol and 2,6-ditert-butyl-para-cresol.

The isocyanate is reacted with the multiple reactive hydrogen compounds such as diols, diamines or triols. In the case of diols or triols, they are typically either polyalkylene ether or polyester polyols. A polyalkylene ether polyol is the preferred active hydrogen containing polymeric material for formulation of the polyurethane. The most useful polyglycols have a molecular weight of 50 to 10,000 and, in the context of the present invention, the most preferred is from about 400 to about 7,000 with the higher molecular weight polyols conferring proportionately greater flexibility upon the polymer. The desired elastomeric behavior will generally require approximately .5-10 percent by weight of a long chain polyol (i.e., 700 to 2,000 eq. wt.) in the polymer.

Examples of the polyether polyols are, but not limited to, polyethylene ether glycol, polypropylene ether glycol, polytetramethylene ether glycol, polyhexamethylene ether glycol, polyoctamethylene ether glycol,

polydecamethylene ether glycol, polydodecamethylene ether glycol, and mixtures thereof. Polyglycols containing several different radicals in the molecular chain, such as, for example, the compound $HO(CH_2OC_2H_4O)_nH$ wherein n is an integer greater than 1, can also be used.

The polyol may also be a hydroxy terminated or hydroxy pendant polyester which can be used instead of or in combination with the polyalkylene ether glycols. Exemplary of such polyesters are those formed by reacting acids, esters or acid halides with glycols. Suitable glycols are polymethylene glycols, such as ethylene, propylene, tetramethylene or decamethylene glycol; substituted methylene glycols such as 2,2-dimethyl-1,3-propane diol, cyclic glycols such as cyclohexane diol and aromatic glycols. Aliphatic glycols are generally preferred when maximized flexibility is desired. The glycols are reacted with aliphatic, cycloaliphatic or aromatic dicarboxylic acids or lower alkyl esters for ester-forming derivatives to produce relatively low molecular weight polymers, preferably having a melting point of less than about 70° C. and a molecular weight comparable to those set forth above for the polyalkylene ether glycols. Acids suitable for use in preparing such polyesters are, for example, phthalic, maleic, succinic, adipic, suberic, sebacic, terephthalic and hexahydrophthalic acids and the alkyl and halogen substituted derivatives of these acids. In addition, a polycaprolactone terminated with hydroxyl groups may also be used.

When used herein, "ionic dispersing agent" means an ionizable acid or base capable of forming a salt with the solubilizing agent. These "ionic dispersing agents" are amines and preferably are water-soluble amines such as triethylamine, tripropylamine, N-ethyl piperidine, and the like; also, acid- and preferably water-soluble acids such as acetic, propionic, lactic, and the like. Naturally, an acid or amine will be selected contingent upon the solubilizing group pendant on the polymer chain.

In forming the polyurethanes useful in the practice of the invention, the polyol and a molar excess of diisocyanate are reacted to form isocyanate terminated polymer. Although suitable reaction conditions and reaction times and temperatures are variable within the context of the particular isocyanate and polyol utilized, those skilled in the art well

recognize the variations. Such skilled artisans recognize that the reactivity of the ingredients involved requires the balance of reaction rate with undesirable secondary reactions leading to color and molecular weight degradation. Typically, the reaction is carried out with stirring at about 50° C. to about 100° C. for about 1 to 4 hours. To provide pendant carboxyl groups, the isocyanate terminated polymer is reacted with a molar deficiency of dihydroxy acid for 1 to 4 hours at 50° C. to 120° C., to form isocyanate prepolymer. The acid is desirably added as a solution, for example, in N-methyl-1,2-pyrrolidone or N-N-dimethylformamide. The solvent for the acid will typically be no more than about 5 percent of the total charge in order to minimize the organic solvent concentration in the polyurethane composition. After the dihydroxy acid is reacted into the polymer chain, the pendant carboxyl groups are neutralized with an amine at about 58-75° C. for about 20 minutes, and chain extension and dispersion are accomplished by addition to water with stirring. A water-soluble diamine may be added to the water as an additional chain extender. The chain extension involves the reaction of the remaining isocyanate groups with water to form urea groups and to polymerize further the polymeric materials, with the result that all the isocyanate groups are reacted by virtue of the addition to a large stoichiometric excess of water.

The dispersion viscosity is generally in the range of from 10 to 1,000 centipoise. Useful solutions of polyurethane in organic solvents, by contrast, generally have viscosities of several thousand centipoise, ranging as high as 50,000 centipoise when the solution contains about 20 to 30 percent by weight polyurethane.

Suitable polyurethane dispersions contain, moreover, about 50 to 75 percent by weight polyurethane solids in dispersion, said solids preferably having a carboxyl content between about 92 and 98 meq per each 100 grams thereof. The preferred polyurethane concentration is 55 to 70 percent by weight and the most preferred concentration is approximately 65 percent by weight polyurethane solids in dispersion.

Particle size, as a useful indicator of dispersion stability, may be measured by light scattering. Useful dispersions having non-settling characteristics will have particles of a diameter of less than 5 microns.

Particularly useful polyurethane dispersions include both the non-crosslinked polyurethane compositions recited in U.S. Patent No. 4,171,391, and the crosslinked polyurethane dispersions of U.S. Patent No. 4,554,308, each of which is incorporated herein by reference. The polyurethane dispersions most preferred for use in the present invention, however, are those available from Witco Chemical Company under the trade designation Witcobond® W-290H. The Witcobond® W-290H dispersions contain 62 percent by weight anionic polyurethane solids having particulate diameters less than 5 microns.

## III. Preparation of the Cohesive Dressing

In order to prepare the cohesive dressing according to the preferred embodiment of the invention, metered amounts of aqueous polyurethane dispersion and aqueous adhesive emulsion are admixed with a stearate salt and a crosslinking agent. Admixing proceeds until the polyurethane dispersion and the adhesive emulsion are substantially uniformly distributed throughout the admixture. The admixture is frothed, cast into a sheet, cured and dried into a self-adherent flexible dressing material in which the polyurethane and adhesive are substantially uniformly distributed throughout.

Suitable stearate salts, or salts of stearic acid, may be selected from the group consisting of aluminum stearate, potassium stearate and sodium stearate. Incorporation of controlled amounts of these stearic acid salts in the polymer/adhesive admixture stabilizes the admixture after frothing, i.e., prevents collapse of the foam prior to or during cure. Cure itself is expedited by the presence of an aziridine crosslinking agent, which crosslinks the frothed polymer upon the application of heat. The aziridine crosslinking agent may be any known aziridine crosslinking agent wherein the agent has monofunctional or polyfunctional aziridine activity due to the incorporation therein of ethyleneimine, propyleneimine, butyleneimine or derivatives thereof. Preferably, the aziridine selection is the polyfunctional aziridine preparation of proprietary formula, sold under the trademark XAMA©-7, which contains 6.35 to 6.95 meq/g aziridine content

and has an aziridine functionality of approximately 3.3. The XAMA®-7 polyfunctional aziridine has a viscosity of 1,200 to 2,000 centipoise at 25° C., further has a density of 1.185 g/cc at 25° C., and is completely miscible with water, acetone, methanol, chloroform and benzene.

The above described admixture of the preferred embodiment of the invention is prepared by combining between 90 and 110 parts by weight of an aqueous ionic polyurethane dispersion containing approximately 62 percent by weight solids, between 20 and 45 parts by weight, and most preferably 35 parts by weight, of an aqueous pressure sensitive adhesive emulsion containing 55 percent by weight solids, between 0.95 and 1.1 parts by weight of XAMA®-7 polyfunctional aziridine and between 10.0 and 20.0, and preferably 15.0, parts by weight of a 33 percent aqueous or nonaqueous dispersion of a stearate salt. Different amounts and concentrations of the constituents of the admixture may be substituted in reactive equivalent amounts. To this admixture may be added additional ingredients and reactive or nonreactive additives, such as surfactants, thickeners, nontoxic absorbent polymeric particles and the like, etc., as desired.

On a laboratory scale, the polyurethane dispersion, adhesive emulsion, stearate and aziridine may be admixed in a Hobart mixer; an Oaks or other industrial frothing mixer is suitable for full scale production. After initial mixing of the ingredients, the admixture is frothed, by agitation and/or inert gas injection, to yield a frothed composition which has very fine, uniform bubbles and which is suitable for immediate casting and curing. Although the froth may be formed into sheets by other means, such as block-foaming and skiving (or slicing), the froth is particularly suited to the knife-casting technique for preparing foam sheet materials. Preferably, therefore, the liquid froth is cast upon a release surface, such as silicone coated release paper, and coated to the desired thickness with, for example, a Gardner knife. The release paper-frothed layer is then passed through an oven to dry and cure the foam. Temperatures between 225° and 350° F. are suitable for initiating the aziridine reaction, curing and drying the foam. The foam may be slightly, partially or completely open-celled as long as it is flexible. The cured foam, in which the polyurethane

and the adhesive are substantially uniformly distributed throughout, preferably has a thickness between 1/6" and 1/8", although thinner and thicker foams demonstrate medical and surgical utility.

The cured and dried sheet is then cut into the desired shapes, packaged, autoclaved or otherwise sterilized, and then distributed for storage or use. Although the cohesive dressing sheets may have any size and shape, the most useful configuration for the cohesive dressing is a rolled tape between 3/4" and about 9" wide, and from about 2 ft. in length. In this configuration, the cohesive dressing may be stored in roll form, cut to the length required and used for wrapping around and covering a limb, the head, the abdomen or chest, or other area of the anatomy.

Although the method described above is the preferred manufacturing technique for the present cohesive dressing, a number of alternate methods are available to prepare the self-adherent foam sheets of the invention. For example, in those applications for which the incorporation of crosslinkers and stabilizers is desired, reactive equivalents of comparable products may be substituted for the aziridine compound and stearic acid salt, respectively. In addition, with vigorous frothing and immediate heat cure, the polyurethane dispersion and adhesive emulsion may be formed into a self-adherent foam sheet without the use of aziridine crosslinking agents or stearate salts at all. Furthermore, although the cohesive dressing is preferably liquid permeable and more preferably is hydrophilic, hydrophobic polyurethane compositions may be used in the admixture, without surfactants, to yield a hydrophobic cohesive dressing having application as an exterior anchor or protective dressing. The cohesive dressing may be prepared with a moisture vapor permeable, moisture impermeable skin, such as a urethane skin, if desired; the urethane skin should contain an amount of the pressure sensitive adhesive solids which corresponds to the amount present in the body of the cohesive dressing. Finally, the cohesive dressing may be prepared with non-polyurethane polymer dispersions as long as such polymer(s) co-cure with the particular pressure sensitive adhesive selected.

The following two examples are illustrative of the preferred embodiment of the invention.

## EXAMPLE I

One hundred parts by weight of Witcobond® W-290H aqueous polyurethane dispersion, containing 62 percent by weight anionic polyurethane solids were admixed, at slow speed in a Hobart mixer, with 17.5 parts by weight of Rhoplex N-560 and 17.5 parts by weight of Rhoplex N-580 pressure sensitive adhesive emulsions, each of which contained 55 percent by weight acrylic adhesive solids. To the admixture was immediately added 15.0 parts by weight of 33 percent aqueous ammonium stearate and 1.0 parts by weight XAMA®-7 polyfunctional aziridine.

The above admixture was frothed in the Hobart mixer 1 minute at low speed, 1 minute at medium speed, 1 minute 15 seconds at high speed, and 2 minutes at low speed. The admixture thus frothed was then coated, at 0.150" gap, over a silicone resin coated release liner. The cast foam was cured and dried by a 15 minute application of heat in a 250° F. oven. The resulting foam layer was porous, self-adherent, nonadherent to dry, healthy skin, and permeable to liquid water.

## EXAMPLE II

The foam layer prepared in accordance with Example I was sliced by automatic rotating knives into cohesive dressing strips measuring 2" in width and 36" in length. The individual strips were wound into separate rolls, packaged in a disposable paper wrapping, and sterilized by microwave irradiation. The foam demonstrated no change in physical properties subsequent to sterilization.

Although the invention has been described with reference to specific materials and specific processes, the invention is to be limited only insofar as is set forth in the accompanying claims.

CLAIMS

1. A cohesive dressing, comprising an integral foam sheet having a polymer and a pressure sensitive adhesive composition therein, whereby said foam sheet is self-adherent and suitable for use as a wound dressing.

2. The cohesive dressing according to claim 1 wherein said polymer and said pressure sensitive adhesive composition are substantially uniformly distributed throughout said foam.

3. The cohesive dressing according to claim 2 wherein said polymer is a polyurethane polymer.

4. The cohesive dressing according to claim 3 wherein said integral foam sheet is liquid permeable.

5. The cohesive dressing according to claim 4 wherein said integral foam sheet is hydrophilic.

6. The cohesive dressing according to claim 4 wherein said integral foam sheet is hydrophobic.

7. The cohesive dressing according to claim 2 wherein said pressure sensitive adhesive composition is selected from the group consisting of acrylic, butadiene-acrylonitrile/phenol-formaldehyde, phenolic, coumarone-indene, polyterpene, polychloroprene/phenol-formaldehyde, and rosin-phenol resins and vinyl alkyl ether and styrene-butadiene adhesives.

8. The cohesive dressing according to claim 7 wherein said pressure sensitive adhesive composition is an acrylic resin.

9. The cohesive dressing according to claim 3 wherein said polyurethane polymer is derived from an aqueous ionically-solubilized-polyurethane dispersion.

10. The cohesive dressing according to claim 8 wherein said acrylic resin is derived from an aqueous acrylic resin emulsion.

11. A method for preparing a cohesive dressing comprising:

a) admixing an aqueous polymeric dispersion with an aqueous emulsion of a pressure sensitive adhesive composition; and

b) frothing, forming, curing and drying the admixture.

12. The method according to claim 11 wherein step a) further comprises the step of:

a) admixing an aqueous ionic polyurethane dispersion with an aqueous emulsion of a pressure sensitive adhesive composition selected from the group consisting of acrylic, butadiene-acrylonitrile/phenol-formaldehyde, phenolic, coumarone-indene, polyterpene, polychloroprene/phenol-formaldehyde, and rosin-phenol resins and vinyl alkyl ether and styrene-butadiene adhesives; and.

13. The method according to claim 12 wherein step a) further comprises the step of:

a) admixing an aqueous ionic polyurethane dispersion with an aqueous emulsion of an acrylic resin pressure sensitive adhesive composition; and.

14. The method according to claim 13 wherein step a) further comprises the step of:

a) admixing an aqueous ionic polyurethane dispersion with an aqueous emulsion of an acrylic resin pressure sensitive adhesive composition and a crosslinking agent until said dispersion and said emulsion are substantially uniformly distributed throughout the admixture; and.

15. The method according to claim 14 wherein step a) further comprises the step of:

a) admixing an aqueous ionic polyurethane dispersion with an aqueous emulsion of an acrylic resin pressure sensitive adhesive composition, along with a crosslinking agent and an aqueous stearate salt, until said dispersion and said emulsion are substantially uniformly distributed throughout .the admixture; and.

16. The method according to claim 13 wherein step b) further comprises the step of:

b) frothing, casting, curing and drying the admixture.

17. The method according to claim 13 wherein step b) further comprises the step of:

b) frothing, casting, heat-curing and drying the admixture.

18. The method according to claim 15 wherein step a) further comprises the step of:

a) admixing 100 parts by weight of an aqueous ionic polyurethane dispersion, 35.0 parts by weight of an acrylic resin pressure sensitive adhesive composition, 1.0 part by weight of an aziridine crosslinking agent and 15 parts by weight of a 33 percent aqueous dispersion of a stearate salt, until said dispersion and said emulsion are substantially uniformly distributed throughout the admixture; and.

0235949

European Patent Office

# EUROPEAN SEARCH REPORT

Application number

EP 87 30 0906

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
| --- | --- | --- | --- |
| X | US-A-4 377 160 (J.W. ROMAINE) * Figures 2-4; column 1, lines 45-57; column 2, lines 17-33; column 3, lines 42-46; column 4, line 64 - column 5, line 23 * --- | 1-7,10 | A 61 F 13/02<br>A 61 L 15/06 |
| X | FR-A-2 093 593 (JOHNSON & JOHNSON) * Page 2, lines 25-38; page 3, lines 33-39; page 4, lines 1-3; page 5, lines 16-22,35-39 * --- | 1-3 | |
| A | FR-A-2 452 290 (KARL OTTO BRAUN KG) * Figures 3,4 * ----- | 1 | |

TECHNICAL FIELDS SEARCHED (Int. Cl.4)

A 61 F
A 61 L

The present search report has been drawn up for all claims

| Place of search | Date of completion of the search | Examiner |
| --- | --- | --- |
| THE HAGUE | 21-05-1987 | ARGENTINI A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document